**Europäisches Patentamt**

(19) **European Patent Office**

**Office européen des brevets**

(11) Publication number : **0 443 532 B1**

(12) # EUROPEAN PATENT SPECIFICATION

(45) Date of publication of patent specification :
29.06.94 Bulletin 94/26

(51) Int. Cl.⁵ : **C07K 7/20, A61K 37/43**

(21) Application number : **91102370.3**

(22) Date of filing : **19.02.91**

(54) **Temporary minimal protection synthesis of LH-RH analogs.**

(30) Priority : **20.02.90 US 482428**

(43) Date of publication of application :
**28.08.91 Bulletin 91/35**

(45) Publication of the grant of the patent :
**29.06.94 Bulletin 94/26**

(84) Designated Contracting States :
**AT BE CH DE DK ES FR GB GR IT LI LU NL SE**

(73) Proprietor : **SYNTEX (U.S.A.) INC.**
**3401 Hillview Avenue**
**Palo Alto California 94304 (US)**

(72) Inventor : **Nestor, John J., Jr.**
**20937 Fairwoods Court**
**Cupertino, CA 95014 (US)**
Inventor : **McClure, Natalie L.**
**17 Coalmine View**
**Portola Valley, CA 94028 (US)**

(74) Representative : **Barz, Peter, Dr. et al**
**Patentanwälte Dipl.-Ing. G. Dannenberg**
**Dr. P. Weinhold, Dr. D. Gudel**
**Dipl.-Ing. S. Schubert, Dr. P. Barz**
**Siegfriedstrasse 8**
**D-80803 München (DE)**

## Description

This invention relates to the solid phase synthesis of LH-RH analog by a minimal protection procedure.

LH-RH analogs are nona- or decapeptides which are structurally related to LH-RH and exhibit biological activity similar to that of LH-RH. The analogs are the subject of intensive clinical investigation due to their demonstrated ability to alleviate the symptoms of endometriosis, prostate cancer, precocious puberty, and other hormonally mediated disorders. While certain LH-RH analogs are currently available for therapeutic use, their synthesis is a complicated and, consequently, expensive procedure which necessarily increases the cost to those in need of treatment. LH-RH analogs are conventionally described as either agonists or antagonists, depending upon their mode of action.

The LH-RH analogs of interest in this invention are nona- and decapeptides, and include both agonists and antagonists. Examples of LHRH agonists useful in the subject invention are nafarelin, leuprorelin, buserelin, goserelin, histerelin, triptorelin and deslorelin; these all differ from naturally occurring LH-RH by replacement of a glycine residue at the 6-position with a D-amino acid. The synthetic agonists then have, in common with the naturally occurring hormone, histidine at position 2, serine at position 4, and tyrosine at position 5, all of which have reactive side chains which may present synthetic difficulties.

The LH-RH antagonists differ from the naturally occurring LH-RH generally by the deletion or replacement of the histidyl residue at position 2. From the synthetic perspective, the deletion of histidine reduces the opportunities for undesired side reactions; however, the presence of both serine and tyrosine still requires that special steps be taken to avoid side chain reactions.

LH-RH analogs may be synthesized by various methods, such as are taught by J.M. Stewart and J.D. Young, Solid Phase Peptide Synthesis, W.H. Freeman Co., San Francisco, 1969; J. Meinenhofer, Hormonal Proteins and Peptides, Vol. 2, page 46, Academic Press (New York), 1973; and E. Schroder and K. Lubke, The Peptides, Vol. 1, Academic Press (New York), 1965. The methods may be broadly characterized as either solution phase or solid phase techniques. Both methods involve the sequential addition of amino acids to a growing peptide chain. Normally, either the amino or carboxyl group of the first amino acid is protected by a suitable protecting group. The protected amino acid can then be either attached to an inert solid support or utilized in solution by adding the next protected amino acid in the sequence under conditions suitable for forming the amide linkage. The protecting group is then removed from this newly added amino acid residue, and the next amino acid is then added and so forth. After all the desired amino acids have been linked in the proper sequence, any remaining protecting groups, and any solid support, are removed to afford the final polypeptide. By simple modification of this general procedure, it is possible to add more than one amino acid at a time to a growing chain, for example, by coupling a protected tripeptide with a protected dipeptide to form a pentapeptide.

The more rigorous conditions of solid phase synthesis, however, generally require that any reactive side chains on the amino acids be protected during formation of the amide linkage. The side chain protecting groups are usually removed in a separate step after cleavage of the completed polypeptide from the inert support on which it is made, or concurrently therewith.

One particularly useful solid phase synthetic method for preparing LH-RH analogs is disclosed in Nestor et al., U.S. Patent No. 4,234,571, the disclosure of which is incorporated by reference herein. In this commonly used approach, the $\alpha$-amino ($N^\alpha$) function of each amino acid is protected by an acid or base sensitive group, such as t-butyloxycarbonyl (Boc); any reactive side chains, as are present on serine, histidine and tyrosine, are also protected with strongly bound groups which require treatment with hydrogen fluoride (HF) or similarly drastic procedures for their removal. Also, the removal of the $\alpha$-amino protecting groups and of the side-chain protecting groups are commonly performed in separate steps.

This approach is adequate for the preparation of research quantities of peptides, but when large scale production of peptides is contemplated, these methods are not satisfactory. Amino acids with fully protected side chains are expensive, and these costs can be significant in a commercial-scale productin of peptides. Also, the use of hydrogen fluoride, in addition to posing serious environmental hazards, contributes to commercially unacceptable yield losses. What is more, because a separate production step is required to remove the side chain protecting groups, this involves additional time and cost in the synthetic process.

Alternate protocols are no more appealing. Tien et al. , Pept. Chem. 375 - 379, T. Shiba and S. Sakakibara (Ed.), Protein Research Foundation, Osaka (1988), have reported the synthesis of LH-RH using tosyl protection on histidine and benzyl protection on tyrosine and serine. This approach, while avoiding the use of hydrogen flouride, still requires a separate dehydrogenation step to remove the benzyl protecting groups, with some reduction of tryptophan occurring.

D.H. Coy et al., Int. J. Peptide Protein Res., 14, 339 - 343 (1979) report the synthesis of the LH-RH antagonist, [D-Phe2, D-Trp3, D-Phe6]-LH-RH using a variety of side chain protection protocols, all of which require HF deprotection: providing benzyl side chain protection of serine only, tosyl side chain protection of arginine only, both serine and arginine side chain protection, and serine, arginine and tyrosine (with 2-bromobenzyl-oxycarbonyl) side chain protection. Salt protection of arginine (as Arg HCl) was also used in the "serine only" synthesis. Hydrogen fluoride was used to cleave the crude peptide from its support and to remove the side chain protecting groups. Only when the peptide is "fully" unprotected (and only salt protection for arginine) did Coy avoid hydrogen fluoride treatment. All of the protected syntheses gave poorer yields than that of the unprotected side chain synthesis.

Coy et al. further reported the synthesis of the LH-RH agonist [D-Leu[6], desGly-NH$_2$[10]]-LH-RH ethylamide with dinitrophenyl side chain protection of histidine only, salt protection of arginine, and no HF treatment. The dinitrophenyl side chain protecting group was removed during cleavage of the peptide from its support with a solution of ethylamine in dimethylformamide. The yield from the histidine-only protected synthesis was only 34% versus 24% for a fully protected, HF cleavage synthesis. No comparison with an unprotected synthesis was made.

The art suggests that, of the various minimal protection strategies, histidine-only protection may provide some improvement in yield over fully protected syntheses for certain LH-RH antagonists; however, no particular benefit is associated with any of the reported side chain protection approaches for LH-RH agonists.

While the ideal approach for eliminating the HF deprotection step may be to conduct an unprotected synthesis, lack of protection for histidine leads to excessive racemization. Following Coy et al., however, we have found that the use of histidine-only protection also results in high levels of a bis-serine impurity, due to the acylation of the serine residue. Significant improvement over the teachings of the art is needed in order to obtain a practicable minimal protection synthesis for LHRH analogs that does not require an HF deprotection step, yet provides protection for those groups which, if unprotected, will adversely affect the purity and yield of peptide.

It is an object of this invention to provide a process for the synthesis of LH-RH analogs in which the side chains of only an essential minimal number of amino acid residues are protected.

It is a further object of this invention to provide a process for the synthesis of LH-RH analogs which obviates the need for an HF deprotection step, and thus also in avoiding the use of the toxic HF reagent, decreasing the toxic waste stream often encountered in conventional processes.

The above noted aspects of the present invention offer the additional advantages of decreasing the costs of preparing the LHRH compounds, as well as avoiding an additional process step to remove the side chain protecting groups.

The objects of this invention are achieved by the process for the solid-phase synthesis of LH-RH and analogs as defined in the claims.

In the temporary minimal protection process of this invention only the hydroxy side chain of the amino acid residue serine is protected with a group which is removed following the coupling of the serine to the peptide chain. The side chain protecting group is one which is labile under the same conditions useful for removing the a-amino protecting group. For those LH-RH analogs which contain a histidine residue, the imidazole side chain may also be protected with a group labile during the coupling cycle, suitably, labile to an $\alpha$-amino group deprotecting agent, but optionally it may also be protected with a group removable by aminolysis or ammonolysis.

Temporary side chain protection of serine and side chain protection of histidine, if present, minimizes formation of impurities and maximizes yields without requiring an HF or alternative separate deprotection step.

Description of the Process and LHRH Analogs

The temporary minimal protection process of this invention is expected to be applicable to the solid phase synthesis of any serine-containing LH-RH analogs. While the invention is described with reference to the sequential addition of individual amino acids, those skilled in the art will recognize that the process is equally applicable to syntheses in which blocks of smaller polypeptides are coupled to form a larger polypeptide, e.g., by adding a tetrapeptide to a pentapeptide, provided that the side chain of any serine residues are temporarily protected during the serine coupling cycle.

Temporary protection means that the serine side chain is protected for a relatively short period of the synthetic cycle. The side chain protecting group and the $\alpha$-amino or carboxyl protecting group are removed simultaneously, after the serine coupling is effected. Generally, the critical criterion for selecting the serine side chain protecting group is that the group be stable to coupling conditions but labile to a-amino deprotecting conditions. In one aspect of this invention, employing $\alpha$-amino protection, the serine side chain is preferably protected by a group selected from t-butyl, t-butyldimethylsilyl, trimethylsilyl, trityl, pivalyl, and tetrahydropyran-2-yl.

For those LH-RH analogs which have histidine residues it is generally desirable to protect the imidazole side chain. This protection may also be of the temporary variety, i.e. labile during the coupling cycle, or may remain in place until the peptide is removed from its support. Preferably, aminolysis or ammonolysis is used to cleave the resin from its support and simultaneously remove the histidine protecting group.

In another aspect of this invention, the deprotecting agent is selected from solutions of hydrogen chloride in $C_3$ to $C_6$ alcohols and dichloromethane. Preferably, the ratio of alcohol to dichloromethane is from 0.1 to 10.0 (v/v) and the acid concentration is 2N to 9N. Most preferably, the alcohol is i-propanol.

## Abbreviations and Definitions

For purposes of this invention, the expression "LH-RH" refers to luteinizing hormone releasing hormone, and "LH-RH analogs" is meant to encompass LH-RH itself as well as other polypeptides that are structurally related to LH-RH or derived from it and that exhibit biological activity similar to that of LH-RH.

The abbreviations for the various common amino acids are those recommended by the IUPAC-IUB Commission on Biochemical Nomenclature, Biochemistry, 11, 1726 (1972). All peptide sequences mentioned herein are written according to the generally accepted convention whereby the N-terminal amino acid is on the left and the C-terminal amino acid is on the right.

The abbreviations herein represent L-amino acids, with the exception of the achiral amino acid glycine, and with the further exception of any unnatural amino acids which are achiral, or are otherwise designated as D- or D,L-. Et is ethyl, Bu is butyl, and iPr is iso-propyl.

Other abbreviations useful in describing the invention involve replacements of the amino acids in the natural LH-RH peptide by the following:

| Amino acid residue | Abbreviation |
|---|---|
| 3-(2-naphthyl)-alanyl | Nal(2) |
| 3-(p-fluorophenyl)-alanyl | p-F-Phe |
| 3-(p-chlorophenyl)-alanyl | p-Cl-Phe |
| 3-(3-pyridyl)-alanyl | Pal(3) |
| $N^G,N^{G'}$-bis(ethyl)-homoarginyl | $hArg(Et)_2$ |
| $N^G,N^{G'}$-bis(2,2,2-trifluoroethyl)-homoarginyl | $hArg(CH_2CF_3)_2$ |
| $N^G$-butyl-homoarginyl | hArg(Bu) |
| $N^\epsilon$-Isopropyl – lysyl | Lys(iPr) |
| (benzyl)-histidyl | His(Bzl) |

As used herein, the term "pharmaceutically acceptable salts" refers to salts that retain the desired biological activity of the parent compound without toxicological side effects. Examples of such salts are acid addition salts formed with inorganic acids, for example hydrochloric acid, hydrobromic acid, sulfuric acid, phosphoric acid, nitric acid and the like; and salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, pamoic acid, alginic acid, polyglutamic acid, naphthalenesulfonic acids, naphthalenedisulfonic acids, polygalacturonic acid, and the like.

The abbreviation "N-Ac" refers specifically to the N-acetyl protecting group, i. e., an acetyl group attached to a terminal amino acid residue on the amine nitrogen, in conformance with generally accepted nomenclature.

## Preferred Embodiments

In one embodiment of the invention there is provided an improved minimal protection process for the solid-phase synthesis of a compound having an amino acid sequence of the formula

$$R^1\text{-}R^2\text{-}R^3\text{-}Ser\text{-}Tyr\text{-}R^4\text{-}Leu\text{-}R^5\text{-}Pro\text{-}R^6 \qquad (1)$$

wherein

$R^1$ is selected from (pyro)Glu and N-Ac-D-Nal(2);

$R^2$ is selected from His, D-p-Cl-Phe and D-p-F-Phe;

$R^3$ is selected from Trp, D-Trp, D-Nal(2) and D-Pal(3);

$R^4$ is selected from D-Nal(2), D-hArg(Et)$_2$, D-hArg(Bu), D-hArg(CH$_2$CF$_3$)$_2$, D-His(Bzl), D-Leu, D-Pal(3), D-Ser(tBu) and D-Trp;

$R^5$ is selected from Arg, L-hArg(Et)$_2$, L-hArg(Bu), L-hArg(CH$_2$CF$_3$)$_2$ and Lys(iPr); and

$R^6$ is selected from Gly-NH$_2$, NH-NHCONH$_2$, D-Ala-NH$_2$ and NHEt;

wherein the amino acids are provided with N$^\alpha$ protection:

which process comprises (a) protecting only the side chain of serine at position 4 with a protecting group labile to those agents useful for removing α-amino protecting groups without inducing racemization, side reactions, or cleavage of the growing peptide from its resin support and (b) protecting the side chain of histidine, if present, with a group labile to an α-amino group deprotection agent, or to aminolysis or ammonolysis, and removing at least the protecting group of the serine side chain immediately after coupling.

In another embodiment, there is provided a temporary minimal protection process for the solid-phase synthesis of a compound of Formula (I), which comprises the following steps: (a) protecting the α-amino groups of the amino acids in the polypeptide, (b) protecting the side chain of serine with a group labile to those agents useful for removing the α-amino protecting group, (c) protecting the side chain of histidine, if present, with a group labile to a basic deprotection agents or a group removable by aminolysis or ammonolysis, (d) bonding the C-terminal amino acid to an inert solid support, (e) sequentially coupling, with a suitable coupling agent, one or more selected amino acids to each other in successive cycles, starting from the C-terminal end, (f) eliminating, at the end of each cycle, the protecting groups by treatment with a deprotecting agent, said deprotecting agent selected from those agents capable of removing both the α-amino protecting group and the side chain protecting group without inducing racemization, side reactions, or cleavage of the growing peptide from the resin, (f) repeating the coupling and eliminating steps as needed to form a nona- or decapeptide, (g) cleaving the polypeptide from the support by aminolysis or ammonolysis, and (h) isolating and purifying the resulting polypeptide.

In yet another embodiment, there is provided a process for the solid-phase synthesis of a compound of Formula (I), which process comprises the steps of:

(a) coupling by solid phase synthesis appropriate Boc-protected amino acids and Boc-protected and t-butyl protected serine in successive cycles and in the order from right to left of the amino acid sequence of the compound of Formula (I), starting with Boc-R6-O- covalently bound to an inert solid support,

(b) eliminating, at the end of each cycle, the Boc-protecting group and simultaneaously the t-butyl group from serine or D-serine by treatment with a deprotecting agent selected from HCl/CH$_2$Cl$_2$ and HCl/lower alkanol/CH$_2$Cl$_2$ to form a polypeptide bound to said solid support,

(c) cleaving the polypeptide from the support by ammonolysis, and

(d) isolating the resulting polypeptide.

In a preferred embodiment, there is provided a process as described above for the production of a polypeptide having the formula above wherein,

$R^1$ is (pyro)Glu or N-Ac-D-Nal(2);

$R^2$ is His or D-p-Cl-Phe;

$R^3$ is Trp and D-Pal(3);

$R^4$ is D-Nal(2), D-Leu, D-Trp, D-Ser(tBu), D-His(Bzl) or D-hArg(Et)$_2$;

$R^5$ is Arg or hArg(Et)$_2$; and

$R^6$ is Gly-NH$_2$, NHEt or D-Ala-NH$_2$.

Most preferably, the invention provides a process for the production of the LH-RH antagonist of Formula (I), i.e., nafarelin, wherein,

$R^1$ is (pyro)Glu,

$R^2$ is His,

$R^3$ is Trp,

$R^4$ is D-Nal(2),

$R^5$ is Arg, and

$R^6$ is Gly-NH$_2$,
or for the LH-RH antagonist of Formula (I) wherein

$R^1$ is Ac-D-Nal(2);
$R^2$ is D-p-Cl-Phe;
$R^3$ is D-Pal(3);
$R^4$ is D-hArg(Et)$_2$;
$R^5$ is L-hArg(Et)$_2$; and
$R^6$ is D-Ala-NH$_2$.

In the preferred embodiment the α-amino (N$^\alpha$) function of the amino acids is protected by an acid or base sensitive group. The protecting group is stable to the conditions of peptide bond formation, while being readily removable without destruction of the growing peptide chain or racemization of any of the chiral centers contained therein. suitable protecting groups are t-butoxycarbonyl (Boc), biphenylisopropyloxycarbonyl, t-amyloxycarbonyl, isobornyloxycarbonyl, α,α-dimethyl-3,5-dimethoxybenzyloxycarbonyl, o-nitrophenylsulfenyl, 2-cyano-t-butyloxycarbonyl, 9-fluorenylmethyloxycarbonyl (Fmoc) and the like. Preferably the α-amino protecting group is t-butoxycarbonyl (Boc). When it is desired to prepare a peptide such as buserelin or goserelin, in which $R^4$ in Formula (I) above, is D-Ser(t-Bu), Fmoc is preferred for N$^\alpha$ protection in the coupling cycles including and following the addition of the D-Ser(tBu).

Fmoc is labile to basic agents (pH > 8.5), such as piperidine, which will not remove tBu from the D-Ser(tBu). In later cycles, following the addition of the D-Ser(tBu), it is required to use base sensitive N$^\alpha$ protection. The side chain of the serine at position 4 is protected with a group which is labile to those agents useful for removing α-amino protecting groups. A preferred serine side chain protecting group is t-butyldimethylsilyl.

The hydroxy side chain of the serine residue is protected during the coupling of serine to the growing peptide, as described for the generic embodiment of this invention. The side chain protecting group is removed after the coupling is effected and prior to adding the next amino acid. The serine side chain protecting group is removed with the same agent used to remove the N$^\alpha$ protecting group. Preferred side chain protecting groups for serine are t-butyl, t-butyldimethylsilyl, trimethylsilyl, trityl, pivalyl and tetrahydropyran-2-yl.

Further, the imidazole side chain of histidine, generally present in LH-RH agonists, is also protected. The histidine side chain protecting group may also be labile during the coupling cycle, for example, labile to an N$^\alpha$ group deprotecting agent, but, optionally, its removal may be completed when the peptide is cleaved from its support. Preferred side chain protecting groups for histidine are p-toluenesulfonyl and 2,4-dinitrophenyl.

To initiate the synthesis, the first amino acid, which will generally be the C-terminal amino acid in the final product, is attached to a suitable solid support. Suitable solid supports useful for the above synthesis are those materials which are inert to the reagents and reaction conditions of the stepwise condensation-deprotection reactions, as well as being insoluble in the media used. Examples of commercially available resins include styrene/divinylbenzene resins modified with a reactive group, e.g., chloromethylated styrene/divinylbenzene copolymer, hydroxymethylated styrene/divinylbenzene copolymer, and the like. Merrifield resin (1% crosslinked chloromethylated styrene/divinylbenzene copolymer) is preferred.

The attachment to the resin, for example, a chloromethylated styrene divinylbenzene resin is made by means of the reaction of the N$^\alpha$ protected C-terminal amino acid, especially the N$^\alpha$-Boc amino acid, as its cesium, tetramethylammonium, triethylammonium, 1,5-diazabicyclo [5.4.0] undec-5-ene, or similar salt in ethanol, acetonitrile, N,N-dimethylformamide (DMF), and the like, especially the cesium salt in DMF, with the chloromethylated resin at an elevated temperature, for example between about 40° and 60° C., preferably about 50° C., for from about 12 to 72 hours, preferably about 48 hours.

The coupling of successive protected amino acids is carried out by methods well known in the art, typically in an automated polypeptide synthesizer. Each protected amino acid is introduced in from about 1.5 to about 2.5-fold molar excess and the coupling is carried out in an inert, non-aqueous, polar solvent such as dichloromethane, DMF or mixtures thereof, preferably in dichloromethane at about ambient temperature. The coupling agent is selected from N,N'-dicyclohexylcarbodiimide (DCC), N,N'-di-iso-propylcarbodiimide (DIC) or other carbodiimide either alone or in the presence of 1-hydroxybenzotriazole (HBt), O-acyl ureas, benzotriazole-Δ-yl- oxy-tris(pyrrolidino phosphonium) hexafluorophosphate (PyBop), N-hydroxysuccinimide, other N-hydroxyimides or oximes. Alternately, protected amino acid active esters (e.g., p-nitrophenyl, pentafluorophenyl and the like) or symmetrical anhydrides may be used.

The peptide resin is checked for complete coupling using the Kaiser Test (Anal. Biochem., 34, 595 (1970)), except for the coupling to proline in which case the Chloranil Test (Anal. Biochem., 117, 145 (1981)) or the Isatin Test (Anal. Chim. Acta, 118, 149 (1980)) is used.

If the completion test(s) suggest that the reaction is not complete, the coupling is repeated using additional amino acid but omitting additional acid deprotection. When the last coupling is completed, the resin is washed with methanol or methanol containing dichloromethane and dried at a maximum of 60°C.

At the end of each cycle, i.e., after each successive $N^\alpha$-protected amino acid is added to the growing polypeptide chain, the protecting group is removed by treatment with a deprotecting agent. When serine is added, the deprotecting agent removes both the $N^\alpha$-Boc protecting group and the serine protecting group. Among the preferred deprotecting agents are hydrogen chloride in dichloromethane ($HCl/CH_2Cl_2$), trifluoroacetic acid in dichloromethane ($TFA/CH_2Cl_2$), and hydrogen chloride dissolved in a C3-C6 alcohol, preferably isopropanol, mixed with dichloromethane. Generally, the concentration of the HCl will be 2N to 9N, preferably 4N to 5N. The ratio of $CH_2Cl_2$ to the C3-C6 alcohol is 0.1 to 10 (v/v), preferably about 1:1. A particularly preferred deprotecting agent is 4.5N HCl in i-PrOH:$CH_2Cl_2$ (1:1). The deprotection step generally takes place at temperatures of 0°C to 45°C, preferably at ambient temperatures (20° C to 27°C).

Those skilled in the art will appreciate that selection of a coupling/deprotection protocol utilizing agents other than those described above is entirely appropriate provided that the serine residue is deprotected with an agent which accomplishes the objectives of this invention. A protocol which uses $HCl/iPrOH/CH_2Cl_2$ for each deprotecting cycle may be employed. Alternatively, a mixed protocol in which $TFA/CH_2Cl_2$ is used for certain cycles and $HCl/iPrOH/CH_2Cl_2$ for others is also useful. Other cycles will be readily apparent to the skilled artisan.

At the end of the solid phase synthesis the polypeptide is cleaved from the resin. Cleavage is by ammonolysis with a saturated solution of ammonia in a suitable solvent for peptides with an alanine or glycine C-terminus; for those peptides having a proline C-terminus cleavage is by means of aminolysis with an alkylamine or fluoroalkylamine. The cleavage is conducted at a temperature between about 10° and 50° C., preferably about 25° C., for between about 12 and 24 hours, preferably about 18 hours. Suitable solvents include methanol, ethanol, isopropanol, dimethylformamide, tetrahydrofuran, N,N-dimethylethanolamine, hexanes and mixtures thereof. Preferably, a saturated solution of ammonia in methanol is used. Alternatively, the peptide may be removed from the resin by transesterification with a base, followed by aminolysis.

The polypeptide is then purified by a sequence of chromatographic steps employing any or all of the following types: ion exchange on a weakly basic resin in the acetate form; hydrophobic adsorption chromatography or underivatized polystyrene-divinylbenzene (e.g, Amberlite® XAD); silica gel adsorption chromatography; ion exchange chromatography on carboxymethylcellulose; partition chromatography (e.g., on Sephadex® G-25), or countercurrent distribution; high performance liquid chromatography (HPLC), especially reversed-phase HPLC on octyl- or octadecylsilyl-silica bonded phase column packing.

If a racemic amino acid is used in one or more of the 1, 2, 3 or 6 positions and individual isomeric products are desired, the diastereomeric nonapeptide or decapeptide final products are separated, and the desired peptide containing a D-amino acid in the appropriate position is isolated and purified, preferably during the above-described chromatographic process.

Optionally, the isolated and purified polypeptide is converted to a pharmaceutically acceptable salt.

The following Examples compare the temporary protection process of this invention with an unprotected process for both an LH-RH agonist and an LH-RH antagonist. These Examples are presented for purposes of specificity only and should not be construed so as to place any undue limitations on the scope of the claimed invention.

In both the products of Examples 1 and 3, using the temporary minimal protection process of this invention, there are significantly fewer impurities compared to the products obtained in Examples 2 and 4 using unprotected syntheses.

In addition to fewer impurities, the process of this invention offers the additional advantages of providing higher yields, and employing less hazardous reagents, over a shorter time period and with lower energy expenditures in the isolation and purification of LH-RH analogs. A further advantage is the generation of smaller amounts of a considerably less toxic waste stream.

PREPARATION A

Preparation of Boc-Gly-O-Resin

4.9 g of $N^\alpha$-Boc-glycine was dissolved in a mixture of 50 ml. methanol and 50 ml. distilled water. The pH of the solution was brought to 7.5 with aqueous cesium bicarbonate. The solvent was then removed under vacuum.

After 18 hours of drying under high vacuum, the residue was dissolved in 150 ml. dry DMF. 25 g 1% chloromethylated polystyrene/divinylbenzene (Merrifield) resin (corresponding to 25 mmole chloride) was added. The mixture was shaken at 50°C for 24 hours, filtered, and the resin was then washed sequentially with DMF, water, and ethanol. The resin was dried under vacuum for 3 days to yield 28.34g of Boc-Gly-O-Resin.

PREPARATION B

Preparation of Boc-Ala-O-Resin

Following the procedures of Preparation A, $N^\alpha$-Boc-D-alanine was added to 1% Merrifield resin to provide $N^\alpha$-Boc-D-Ala-O-resin.

EXAMPLE 1

SYNTHESIS OF NAFARELIN WITH TEMPORARY SERINE PROTECTION

In this Example, nafarelin was prepared using the following side chain protection protocol: salt protection for arginine (as the chloride), tosyl protection for histidine, and t-butyl protection for serine.

$N^\alpha$-Boc amino acids were obtained from Bachem (Torrance, CA) (Leu, Tyr, His(Tos), Arg, Trp and Gly), Star Biochemicals (Torrance, CA) (Pro and Ser(tBu)), Synthe Tech (Albany, OR) (D-Nal(2)).

Solutions of 4-4.5N HCl in i-PrOH/$CH_2Cl_2$(1/1) were prepared by bubbling HCl into cooled i-PrOH. Once the solution became saturated (determined by titration, approximately 9N), the solution was kept at room temperature for no more than 3 days and diluted with an equal volume of $CH_2Cl_2$ before use.

1.0 mmol of $N^\alpha$-Boc-Gly-O-resin from Preparation A was placed in the reaction vessel of a 5.0 L Vega 296 automated solid phase peptide synthesizer fitted with accessory bottles and flasks for addition of reagents and for pressurization, depressurization and maintenance of an inert atmosphere of nitrogen.

The following amino acids were added to the $N^\alpha$-Boc-Gly-O-resin by DIC or HBt-assisted DIC coupling for 3 hours:

$N^\alpha$-Boc-Pro 2.0 equiv.
$N^\alpha$-Boc-Arg.HCl 2.0 equiv.
$N^\alpha$-Boc-Leu.$H_2O$ 2.0 equiv.
$N^\alpha$-Boc-D-Nal(2) 1.5 equiv./HBt
$N^\alpha$-Boc-Tyr 1.5 equiv./HBt
$N^\alpha$-Boc-Ser(tBu) 2.0 equiv./HBt
$N^\alpha$-Boc-Trp 1.75 equiv./HBt
$N^\alpha$-Boc-His(Tos) 1.75 equiv./HBt
(pyro)Glu 2.5 equiv./HBt

The following protocols were used to remove the $N^\alpha$ protecting group following each addition.

Program A: The resin was first washed with $CH_2Cl_2$ 1x1min., TFA-$CH_2Cl_2$ (40/60) 1x1min., TFA-$CH_2Cl_2$ (40/60) 1x30 min., $CH_2Cl_2$ 5x1min., $Et_3N$-$CH_2Cl_2$(5/95) 3x1min., $CH_2Cl_2$ 4x1min.

Program B: The resin was first washed with $CH_2Cl_2$ 1x1min., 4-4.5N HCl in $CH_2Cl_2$/i-PrOH(1/1) 1x1min., 4-4.5N HCl in $CH_2Cl_2$/i-PrOH (1/1) 1x30min., $CH_2Cl_2$ 3x1min., DMF 1x1min., $Et_3N$-$CH_2Cl_2$(5/95) 3x1min., DMF 1x1min., $CH_2Cl_2$ 4x1min.

Program A was used to remove the $N^\alpha$ protecting groups on Gly, Pro, Arg, Leu, D-Nal(2) and Tyr. Program B was used for the removal of the $N^\alpha$ protecting groups on Ser, Trp, and His and for the removal of the serine side chain protecting group.

After each deprotecting and washing step, following protocol A or B, the next amino acid in sequence was added and the resin washed with $CH_2Cl_2$ 3x1min., MeOH 4x1min., DMF 2x1min. and $CH_2Cl_2$ 4x1min. When the sequence was completed, the peptide was cleaved from the resin by treatment with a saturated solution of ammonia in methanol for about 18 hours at about 25°C.

The crude peptide was dissolved in 2M acetic acid and converted to the acetate salt by passage through a column of AG3-X4A resin (Bio-Rad). The acetate was dissolved in a minimal amount of methanol and acetone added to reprecipitate the peptide. Reversed phase HPLC (Partisil ODS-3, 40μ, acetonitrile with 0.5% acetic acid) was used to remove polar and non-polar impurities. Fractions containing at least 97% nafarelin acetate were combined and diluted with water and reloaded on a reversed phase HPLC column, and washed with 1% acetic acid water. The residue was precipitated, filtered, washed and then dried under vacuum.

Amino acid analyses were performed on a Beckman 119CL amino acid analyzer. Samples for amino acid analyses were hydrolyzed with 4N $CH_3SO_3H$ (0.2% 3-(2-aminomethyl indole) HCl) for 20 hrs at 110°C.

Analytical HPLC was performed on a Spectra Physics 8800 chromatograph, using an ODS-II column from Alltech, 5μ, 4.6 x 250mm, 10 μl inj., flow - 1.5 ml/min., 27.5% $CH_3CN$, 72.5% 0.16M $KH_2PO_4$ pH=5.1, temp. =40°C.

HPLC analysis of the crude peptide showed a main peak with a retention time of 18 min. corresponding to nafarelin and no impurity over 1% at rt 14 min.

## EXAMPLE 2

### SYNTHESIS OF NAFARELIN WITHOUT SERINE PROTECTION

The procedure of Example 1 was followed except that $N^\alpha$-Boc-Ser was substituted for $N^\alpha$-Boc-Ser(tBu).

HPLC analysis showed a main peak at 18 min. corresponding to nafarelin and 8.1 to 11.5% of an impurity at a retention time (rt) of 14 min.

Also, the yield from this "unprotected" synthesis was approximately the same as that obtained from a fully protected synthesis with an HF treatment; in the latter instance, the yield was significantly lower than that achieved with the temporary protection synthesis of Example 1.

## EXAMPLE 3

### SYNTHESIS OF AN LH-RH ANTAGONIST USING TEMPORARY SERINE PROTECTION

In this Example an LH-RH antagonist, $N$-Ac-D-Nal(2)-D-pCl-Phe-D-Pal(3)-Ser-Tyr-D-hArg(Et)$_2$-Leu-hArg(Et)$_2$-Pro-D-AlaNH$_2$, was prepared using the following side chain protection protocol: salt protection for L- and D-hArg(Et)$_2$ (as the chloride) and t-butyl protection for serine.

$N^a$-Boc amino acids were obtained from Bachem (Torrance, CA) (D-Ala, Arg and Leu); Star Biochemicals (Torrance, CA) (Pro); Synthe Tech (Albany, OR) (D-Nal(2)), Incell (Milwaukee, WI) (D-Pal(3)) and UCB Bioproducts (Belgium)(p-Cl-Phe).

Amino acids were added to the $N^\alpha$-Boc-D-Ala-O-Resin of Preparation B in the following sequence:

$N^\alpha$-Boc-Pro 2.3 equiv.

$N^\alpha$-Boc-hArg(Et)$_2$.HCl 1 equiv./HBt

$N^\alpha$-Boc-Leu.H$_2$O 2.3 equiv.

$N^\alpha$-Boc-D-hArg(Et)$_2$.HCl 1.6 equiv./HBt

$N^\alpha$-Boc-Tyr 2.1 equiv./HBt

$N^\alpha$-Boc-Ser(tBu) 2.0 equiv.

$N^\alpha$-Boc-D-Pal(3) 1.8 equiv./HBt

$N^\alpha$-Boc-D-p-Cl-Phe 2.0 equiv.

$N^\alpha$-Boc-D-Nal(2) 2.1 equiv./HBt

Acetic anhydride

An acetylation (capping) was done after Ala, Pro and Leu. Excess HBt (2 equiv.) was used for the coupling of the basic amino acids, hArg(Et)$_2$ and Pal(3).

The amino acids were attached by DIC or HBt-assisted DIC coupling for 3 hours and the resin was subsequently washed with CH$_2$Cl$_2$ 3x1min., MeOH 4x1min., DMF 2x1min. and CH$_2$Cl$_2$ 4x1min.

The following protocols were used to remove the $N\alpha^a$ protecting group following each addition:

Program A: The resin was first washed with CH$_2$Cl$_2$ 1x1min., TFA-CH$_2$Cl$_2$ (40/60) 1x1min., TFA-CH$_2$Cl$_2$ (40/60) 1x30 min., CH$_2$Cl$_2$ 5x1min., Et$_3$N-CH$_2$Cl$_2$(5/95) 3x1min., CH$_2$Cl$_2$ 4x1min.

Program B: The resin was first washed with CH$_2$Cl$_2$ 1x1min., 4-4.5N HCl in CH$_2$Cl$_2$/i-PrOH(1/1) 1x1min., 4-4.5N HCl in CH$_2$Cl$_2$/i-PrOH (1/1) 1x30min., CH$_2$Cl$_2$ 3x1min., DMF 1x1min., Et$_3$N-CH$_2$Cl$_2$(5/95) 3x1min., DMF 1x1min., CH$_2$Cl$_2$ 4x1min.

Program A was used for the removal of the protecting groups on Ala, Pro, L-hArg(Et)$_2$, Leu and D-Nal(2); Program B was used for the removal of the protecting groups on D-hArg(Et)$_2$, Tyr, Ser, D-Pal(3) and p-Cl-Phe.

After each deprotecting and washing step, following protocol A or B, the next amino acid in sequence was added and the resin washed with CH$_2$Cl$_2$ 3x1min., MeOH 4x1min., DMF 2x1min. and CH$_2$Cl$_2$ 4x1min. When the sequence was completed, the peptide was cleaved from the resin by treatment with a saturated solution of ammonia in methanol for about 18 hours at about 25°C.

The crude peptide was first dissolved in 2M acetic acid and converted to its acetate salt by passage through a column of AG3-X4A resin (Bio-Rad). The acetate was subjected to chromatography on a silica gel column (CH$_2$Cl$_2$/i-PrOH/MeOH/H$_2$O/HOAc solvent); the acetate fractions dissolved in water and loaded onto a reversed-phase column (Vydec c-18, 15-20µ) and purified using acetonitrile(TEAP (pH 3). Fractions of the desired purity were combined and diluted with water and reloaded on a reversed-phase HPLC column, then washed with 1% acetic acid in water. The peptide was stripped with a mixture of MeOH/CH$_3$CN/HOAc/H$_2$O (44/50/1/5). The residue was dissolved in methanol or acetic acid and precipitated over ether, filtered, washed with ether and dried under vacuum.

Amino acid analyses were performed on a Beckman 119CL amino acid analyzer. Samples for amino acid analyses were hydrolyzed with 6N HCl at 110° C for 20 hrs.

Analytical HPLC was performed on a Spectra Physics 8800 chromatograph, using a Spherisorb C-8 (Alltech), $5\mu$, 4.6 x 250 mm, $10\mu l$ inj., flow =1.5 ml/min., 30% $CH_3CN$, 70% $NH_4H_2PO_40.04M$, dimethyloctylamine 4.3 x $10^{-3}$, temp. 40° C.

Synthesis of the antagonist was confirmed by the presence of a main peak at rt 18 min.; no other peak over 1% was noted at rt 16 min.


## EXAMPLE 4

### SYNTHESIS OF LH-RH ANTAGONIST WITHOUT TEMPORARY SERINE PROTECTION

Example 3 was repeated using $N^\alpha$-Boc-Ser instead of $N^a$-Boc-Ser(tBu).

HPLC analysis showed the presence of a main peak at 18 min. corresponding to the antagonist and the presence of an impurity of 6.5% at rt 16 min.


## Claims

1. A process for the solid phase synthesis of an LH-RH analog containing at least one serine residue, which process comprises protecting only the side chain of the serine residue with a protecting group which is labile to those agents useful for removing $\alpha$-amino protecting groups and removing said protecting group after coupling.

2. A process of claim 1 in which the serine side chain is protected with a group selected from t-butyl, trityl, pivalyl, t-butyldimethylsilyl, trimethylsilyl, and tetrahydropyranyl.

3. A process of claim 2 in which the serine side chain protecting group is removed by treatment with hydrogen chloride in a C3-C6 alcohol / dichloromethane solution.

4. A process of claim 3 in which the C3-C6 alcohol is isopropanol.

5. A process for the solid-phase synthesis of a compound having an amino acid sequence of the formula
$$R^1\text{-}R^2\text{-}R^3\text{-}Ser\text{-}Tyr\text{-}R^4\text{-}Leu\text{-}R^5\text{-}Pro\text{-}R^6 \qquad (I)$$
wherein

$R^1$ is selected from (pyro)Glu and N-Ac-D-Nal(2);

$R^2$ is selected from His, D-p-Cl-Phe and D-p-F-Phe;

$R^3$ is selected from Trp, D-Trp, D-Nal(2) and D-Pal(3);

$R^4$ is selected from D-Nal(2), D-hArg(Et)$_2$, D-hArg(Bu), D-hArg(CH$_2$CF$_3$)$_2$, D-His(Bzl), D-Leu, D-Pal(3), D-Ser(tBu) and D-Trp;

$R^5$ is selected from Arg, L-hArg(Et)$_2$, L-hArg(Bu), L-hArg(CH$_2$CF$_3$)$_2$ and Lys(iPr); and

$R^6$ selected from Gly-NH$_2$, NH-NHCONH$_2$, D-Ala-NH$_2$ and NHEt;

wherein the amino acids are provided with $N^\alpha$ protection;

which process comprises (a) protecting only the side chain of serine at position 4 with a protecting group which is labile to those agents useful for removing a-amino protecting groups and (b) protecting the side chain of histidine, if present, with a group labile to an $\alpha$-amino group deprotection agent, or to aminolysis or ammonolysis, and removing at least the protecting group of the serine side chain after coupling.

6. A process of Claim 5 in which the serine side chain protecting group is selected from t-butyl, trityl, pivalyl, tetrahydropyran-2-yl, trimethylsilyl and t-butyldimethylsilyl, preferably, t-butyl.

7. A process of Claim 5 in which the $\alpha$-amino protecting group is selected from t-butyloxycarbonyl, t-amyloxycarbonyl, isobornyloxycarbonyl, $\alpha,\alpha$-dimethyl-3,5-dimethoxybenzyloxycarbonyl, o-nitrophenylsulfenyl, 2-cyano-t-butyloxycarbonyl, and 9-fluorenylmethyloxycarbonyl.

8. A process of claim 5 in which the $\alpha$-amino protecting group is t-butyloxycarbonyl, the serine side chain protecting group is t-butyl, and the histidine side chain protecting group is p-toluenesulfonyl.

9. A process of any one of Claims 1-8 in which the deprotecting agent is selected from HCl/CH$_2$Cl$_2$, TFA/CH$_2$Cl$_2$, and HCl/(C3-C6)alcohol/CH$_2$Cl$_2$.

10. A process of Claim 9 in which the deprotecting agent is $HCl/iPrOH/CH_2Cl_2$.

11. A process of any one of Claims 5-10 in which

$R^1$ is Ac-D-Nal(2);
$R^2$ is D-p-Cl-Phe;
$R^3$ is D-Pal(3);
$R^4$ is D-hArg(Et)$_2$;
$R^5$ is L-hArg(Et)$_2$; and
$R^6$ is D-Ala-NH$_2$.

12. A process of any one of Claims 5-10 in which

$R^1$ is (pyro)Glu,
$R^2$ is His,
$R^3$ is Trp,
$R^4$ is D-Nal(2),
$R^5$ is Arg, and
$R^6$ is Gly-NH$_2$.

13. A process for the solid-phase synthesis of a compound having an amino acid sequence of the formula

$$R^1\text{-}R^2\text{-}R^3\text{-Ser-Tyr-}R^4\text{-Leu-}R^5\text{-Pro-}R^6 \qquad (I)$$

wherein
$R^1$ is selected from (pyro)Glu and N-Ac-D-Nal(2);
$R^2$ is selected from His, D-p-Cl-Phe and D-p-F-Phe;
$R^3$ is selected from Trp, D-Trp, D-Nal(2) and D-Pal(3);
$R^4$ is selected from D-Nal(2), D-hArg(Et)$_2$, D-hArg(Bu), D-hArg(CH$_2$CF$_3$)$_2$, D-His(Bzl), D-Leu, D-Pal(3), D-Ser(tBu) and D-Trp;
$R^5$ is selected from Arg, L-hArg(Et)$_2$, L-hArg(Bu), L-hArg(CH$_2$CF$_3$)$_2$ and Lys(iPr); and
$R^6$ is selected from Gly-NH$_2$, NH-NHCONH$_2$, D-Ala-NH$_2$ and NHEt;
which process comprises the steps of:
(a) attaching Boc-R6-O to an inert solid support;
(b) adding appropriate Boc protected amino acids and the serine protected by both Boc and t-butyl, in successive cycles and in the order from right to left of the amino acid sequence of the compound of Formula (I),
(c) eliminating, at the end of each cycle, the Boc-protecting group and simultaneously the t-butyl group from the serine or D-serine by treatment with a deprotecting agent selected from HCl/CH$_2$Cl$_2$ and HCl/lower alkanol/CH$_2$Cl$_2$ to form a polypeptide bound to said solid support,
(d) cleaving the polypeptide from the support by ammonolysis, and
(e) isolating the resulting polypeptide.

**Patentansprüche**

1. Verfahren zur Festphasen-Synthese eines LH-RH-Analogons, das mindestens einen Serinrest enthält, umfassend das Schützen lediglich der Seitenkette des Serinrestes mit einer Schutzgruppe, die gegenüber denjenigen Mitteln labil ist, die zum Entfernen von $\alpha$-Amino-Schutzgruppen nützlich sind, und das Entfernen der Schutzgruppe nach Kuppeln.

2. Verfahren nach Anspruch 1, in dem die Serin-Seitenkette mit einer aus t-Butyl, Trityl, Pivalyl, t-Butyldimethylsilyl, Trimethylsilyl und Tetrahydropyranyl ausgewählten Gruppe geschützt wird.

3. Verfahren nach Anspruch 2, in dem die Serin-Seitenkettenschutzgruppe durch Behandlung mit Chlorwasserstoff in einer C3-C6-Alkohol/Dichlormethan-Lösung entfernt wird.

4. Verfahren nach Anspruch 3, in dem der C3-C6-Alkohol Isopropanol ist.

5. Verfahren zur Festphasen-Synthese einer Verbindung mit einer Aminosäuresequenz der Formel

$$R^1\text{-}R^2\text{-}R^3\text{-Ser-Tyr-}R^4\text{-Leu-}R^5\text{-Pro-}R^6 \qquad (I)$$

worin
$R^1$ ausgewählt ist aus (pyro)Glu und N-Ac-D-Nal(2);

$R^2$ ausgewählt ist aus His, D-p-Cl-Phe und D-p-F-Phe;
$R^3$ ausgewählt ist aus Trp, D-Trp, D-Nal(2) und D-Pal(3);
$R^4$ ausgewählt ist aus D-Nal(2), D-hArg(Et)$_2$, D-hArg(Bu), D-hArg(CH$_2$CF$_3$)$_2$, D-His(Bzl), D-Leu, D-Pal(3), D-Ser(tBu) und D-Trp;
$R^5$ ausgewählt ist aus Arg, L-hArg(Et)$_2$, L-hArg(Bu), L-hArg(CH$_2$CF$_3$)$_2$ und Lys(iPr); und
$R^6$ ausgewählt ist aus Gly-NH$_2$, NH-NHCONH$_2$, D-Ala-NH$_2$ und NHEt;
worin die Aminosäuren mit N$^\alpha$-Schutz versehen sind;
umfassend (a) das Schützen lediglich der Seitenkette von Serin in der Stellung 4 mit einer Schutzgruppe, die gegenüber denjenigen Mitteln labil ist, die zum Entfernen von $\alpha$-Amino-Schutzgruppen nützlich sind, und (b) das Schützen der Seitenkette von Histidin, falls anwesend, mit einer Gruppe, die gegenüber einem Mittel, das eine $\alpha$-Aminogruppe Schutzgruppe entfernt, oder gegen Aminolyse oder Ammonolyse labil ist, und das Entfernen mindestens der Schutzgruppe der Serin-Seitenkette nach Kuppeln.

6. Verfahren nach Anspruch 5, in dem die Serin-Seitenkettenschutzgruppe ausgewählt ist aus t-Butyl, Trityl, Pivalyl, Tetrahydropyran-2-yl, Trimethylsilyl und t-Butyldimethylsilyl, vorzugsweise t-Butyl.

7. Verfahren nach Anspruch 5, in dem die $\alpha$-Amino-Schutzgruppe ausgewählt ist aus t-Butyloxycarbonyl, t-Amyloxycarbonyl, Isobornyloxycarbonyl, $\alpha,\alpha$-Dimethyl-3,5-dimethoxybenzyloxycarbonyl, o-Nitrophenylsulfenyl, 2-Cyano-t-butyloxycarbonyl und 9-Fluorenylmethyloxycarbonyl.

8. Verfahren nach Anspruch 5, in dem die $\alpha$-Amino-Schutzgruppe t-Butyloxycarbonyl ist, die Serin-Seitenkettenschutzgruppe t-Butyl ist und die Histidin-Seitenkettenschutzgruppe p-Toluolsulfonyl ist.

9. Verfahren nach irgendeinem der Ansprüche 1 - 8, in dem das Schutzgruppen entfernende Mittel ausgewählt ist aus HCl/CH$_2$Cl$_2$, TFA/CH$_2$Cl$_2$ und HCl/(C3-C6)-Alkohol/CH$_2$Cl$_2$.

10. Verfahren nach Anspruch 9, in dem das Schutzgruppen entfernende Mittel HCl/iPrOH/CH$_2$Cl$_2$ ist.

11. Verfahren nach irgendeinem der Ansprüche 1 - 10, in dem
R$^1$ Ac-D-Nal(2) ist;
R$^2$ D-p-Cl-Phe ist;
R$^3$ D-Pal(3) ist;
R$^4$ D-hArg(Et)$_2$ ist;
R$^5$ L-hArg(Et)$_2$ ist; und
R$^6$ D-Ala-NH$_2$ ist.

12. Verfahren nach irgendeinem der Ansprüche 5 - 10, in dem
R$^1$ (pyro)Glu ist;
R$^2$ His ist;
R$^3$ Trp ist;
R$^4$ D-Nal(2) ist;
R$^5$ Arg ist; und
R$^6$ Gly-NH$_2$ ist.

13. Verfahren zur Festphasen-Synthese einer Verbindung mit einer Aminosäuresequenz der Formel
$$R^1\text{-}R^2\text{-}R^3\text{-Ser-Tyr-}R^4\text{-Leu-}R^5\text{-Pro-}R^6 \qquad (I)$$
worin
R$^1$ ausgewählt ist aus (pyro)Glu und N-Ac-D-Nal(2);
R$^2$ ausgewählt ist aus His, D-p-Cl-Phe und D-p-F-Phe;
R$^3$ ausgewählt ist aus Trp, D-Trp, D-Nal(2) und D-Pal(3);
R$^4$ ausgewählt ist aus D-Nal(2), D-hArg(Et)$_2$, D-hArg(Bu), D-hArg(CH$_2$CF$_3$)$_2$, D-His(Bzl), D-Leu, D-Pal(3), D-Ser(tBu) und D-Trp;
R$^5$ ausgewählt ist aus Arg, L-hArg(Et)$_2$, L-hArg(Bu), L-hArg(CH$_2$CF$_3$)$_2$ und Lys(iPr); und
R$^6$ ausgewählt ist aus Gly-NH$_2$, NH-NHCONH$_2$, D-Ala-NH$_2$ und NHEt;
umfassend die Schritte:
(a) das Anbringen von Boc-R6-O an einen inerten festen Träger;
(b) das Hinzufügen von geeigneten Boc-geschützten Aminosäuren und dem Serin, das sowohl durch Boc als auch durch t-Butyl geschützt ist, in aufeinanderfolgenden Zyklen und in der Reihenfolge von rechts nach links der Aminosäuresequenz der Verbindung der Formel (I),

(c) das Eliminieren der Boc-Schutzgruppe und gleichzeitig der t-Butylgruppe aus dem Serin oder D-Serin am Ende jedes Zyklus durch Behandeln mit einem Schutzgruppen entfernenden Mittel, das aus HCl/ $CH_2Cl_2$ und HCl/Niederalkanol/$CH_2Cl_2$ ausgewählt ist, um ein Polypeptid zu bilden, das an den festen Träger gebunden ist,

(d) das Abspalten des Polypeptids vom Träger durch Ammonolyse und

(e) das Isolieren des resultierenden Polypeptids.

**Revendications**

1. Procédé de synthèse en phase solide d'un analogue de LH-RH contenant au moins un résidu sérine, procédé qui comprend l'étape consistant à protéger seulement la chaîne latérale du résidu sérine avec un groupe protecteur qui est labile vis-à-vis des agents utiles pour l'élimination des groupes protecteurs de la fonction α-amino, et, après couplage, l'élimination dudit groupe protecteur,

2. Procédé suivant la revendication 1, dans lequel la chaîne latérale de la sérine est protégée avec un groupe choisi entre les groupes tertio-butyle, trityle, pivalyle, tertio-butyldiméthylsilyle, triméthylsilyle et tétrahydropyrannyle.

3. Procédé suivant la revendication 2, dans lequel le groupe protecteur de la chaîne latérale de la sérine est éliminé par traitement avec une solution de chlorure d'hydrogène dans un mélange alcool en C3 à C6/dichlorométhane.

4. Procédé suivant la revendication 3, dans lequel l'alcool en C3 à C6 est l'isopropanol.

5. Procédé de synthèse en phase solide d'un composé possédant une séquence d'amino-acides de formule

$$R^1\text{-}R^2\text{-}R^3\text{-}Ser\text{-}Tyr\text{-}R^4\text{-}Leu\text{-}R^5\text{-}Pro\text{-}R^6 \qquad (I)$$

dans laquelle

$R^1$ est choisi entre les groupes (pyro)Glu et N-Ac-D-Nal(2) ;

$R^2$ est choisi entre les groupes His, D-p-Cl-Phe et D-p-F-Phe ;

$R^3$ est choisi entre les groupes Trp, D-Trp, D-Nal(2) et D-Pal(3) ;

$R^4$ est choisi entre les groupes D-Nal(2), D-hArg(Et)$_2$, D-hArg(Bu), D-hArg($CH_2CF_3$)$_2$, D-His(Bzl), D-Leu, D-Pal(3), D-Ser(tBu) et D-Trp ;

$R^5$ est choisi entre Arg, L-hArg(Et)$_2$, L-hArg(Bu), L-hArg($CH_2CF_3$)$_2$ et Lys(iPr) ; et

$R^6$ est choisi entre Gly-NH$_2$, NH-NHCONH$_2$, D-Ala-NH$_2$ et NHEt ;

les amino-acides étant munis d'une protection sur l'atome Nα ;

procédé qui comprend (a) l'étape consistant à protéger seulement la chaîne latérale de la sérine en position 4 avec un groupe protecteur qui est labile vis-à-vis des agents utiles pour l'élimination des groupes protecteurs de la fonction α-amino, et (b) la protection de la chaîne latérale de l'histidine, si elle est présente, avec un groupe labile vis-à-vis d'un agent d'élimination de la protection d'un groupe α-amino, d'une aminolyse ou ammonolyse, et, après couplage, l'élimination d'au moins le groupe protecteur de la chaîne latérale de la sérine.

6. Procédé suivant la revendication 5, dans lequel le groupe protecteur de la chaîne latérale de la sérine est choisi entre les groupes tertio-butyle, trityle, pivalyle, tétrahydropyranne-2-yle, triméthylsilyle et tertio-butyldiméthylsilyle, et consiste de préférence en le groupe tertio-butyle.

7. Procédé suivant la revendication 5, dans lequel le groupe protecteur de la fonction α-amino est choisi entre les groupes tertio-butyloxycarbonyle, tertio-amyloxycarbonyle, isobornyloxycarbonyle, α,α-diméthyl-3,5-diméthoxybenzyloxycarbonyle, ortho-nitrophénylsulfényle, 2-cyano-tertio-butyloxycarbonyle et 9-fluorénylméthyloxycarbonyle.

8. Procédé suivant la revendication 5, dans lequel le groupe protecteur de la fonction α-amino est un groupe tertio-butyloxycarbonyle, le groupe protecteur de la chaîne latérale de la sérine est un groupe tertio-butyle, et le groupe protecteur de la chaîne latérale de l'histidine est un groupe p-toluènesulfonyle.

9. Procédé suivant l'une quelconque des revendications 1 à 8, dans lequel l'agent d'élimination de protection est choisi entre un mélange HCl/$CH_2Cl_2$, un mélange TFA/$CH_2Cl_2$ et un mélange HCl/alcool en C3 à C6/$CH_2Cl_2$.

**10.** Procédé suivant la revendication 9, dans lequel l'agent d'élimination de protection est un mélange HCl/iPrOH/CH$_2$Cl$_2$.

**11.** Procédé suivant l'une quelconque des revendications 5 à 10, dans lequel
R$^1$ représente un groupe Ac-D-Nal(2) ;
R$^2$ représente un groupe D-p-Cl-Phe ;
R$^3$ représente un groupe D-Pal(3) ;
R$^4$ représente un groupe D-hArg(Et)$_2$ ;
R$^5$ représente un groupe L-hArg(Et)$_2$ ; et
R$^6$ représente un groupe D-Ala-NH$_2$.

**12.** Procédé suivant l'une quelconque des revendications 5 à 10, dans lequel
R$^1$ représente un groupe (pyro)Glu,
R$^2$ représente un groupe His,
R$^3$ représente un groupe Trp,
R$^4$ représente un groupe D-Nal(2),
R$^5$ représente un groupe Arg, et
R$^6$ représente un groupe Gly-NH$_2$.

**13.** Procédé de synthèse en phase solide d'un composé possédant une séquence d'amino-acides de formule
R$^1$-R$^2$-R$^3$-Ser-Tyr-R$^4$-Leu-R$^5$-Pro-R$^6$    (I)
dans laquelle
R$^1$ est choisi entre les groupes (pyro)Glu et N-Ac-D-Nal(2) ;
R$^2$ est choisi entre les groupes His, D-p-Cl-Phe et D-p-F-Phe ;
R$^3$ est choisi entre les groupes Trp, D-Trp, D-Nal(2) et D-Pal(3) :
R$^4$ est choisi entre les groupes D-Nal(2), D-hArg(Et)$_2$, D-hArg(Bu), D-hArg(CH$_2$CF$_3$)$_2$, D-His(Bzl), D-Leu, D-Pal(3), D-Ser(tBu) et D-Trp ;
R$^5$ est choisi entre les groupes Arg, L-hArg(Et)$_2$, L-hArg(Bu), L-hArg(CH$_2$CF$_3$)$_2$ et Lys(iPr) ; et
R$^6$ est choisi entre les groupes Gly-NH$_2$, NH-NHCONH$_2$, D-Ala-NH$_2$ et NHEt ;
procédé qui comprend les étapes :
(a) de fixation de Boc-R6-O à un support solide inerte ;
(b) d'addition d'amino-acides appropriés protégés avec un groupe Boc et de la sérine protégée à la fois avec un groupe Boc et un groupe tertio-butyle, dans des cycles successifs et dans l'ordre de la droite vers la gauche de la séquence d'amino-acides du composé de formule (I),
(c) d'élimination, à la fin de chaque cycle, du groupe protecteur Boc et, simultanément, du groupe tertiobutyle de la sérine ou de la D-sérine par traitement avec un agent d'élimination de protection choisi entre un mélange HCl/CH$_2$Cl$_2$ et un mélange HCl/alcanol inférieur/CH$_2$Cl$_2$ pour former une liaison polypeptidique avec ledit support solide,
(d) de clivage du polypeptide du support par ammonolyse, et
(e) d'isolement du polypeptide résultant.